# EUROPEAN PATENT SPECIFICATION

(11) **EP 3 310 360 B1**
(45) Date of publication and mention of the grant of the patent: **27.03.2024**
(21) Application number: 16812614.2
(22) Date of filing: 20.06.2016
(51) Int. Cl.: C07K 5/06, C07K 5/078, C07K 4/00, C07F 5/02, A61K 38/00

(54) **CHIRAL SPECIFIC BORON-CONTAINING COMPOUNDS AND THEIR USE IN TREATING CANCER OR AMYLOIDOSIS**
CHIRALE SPEZIFISCHE BORHALTIGE VERBINDUNGEN UND DEREN VERWENDUNG IN DER BEHANDLUNG VON KREBS ODER AMYLOIDOSE
COMPOSÉS SPÉCIFIQUES CHIRAUX À BASE DE BORE ET LEUR UTILISATION DANS LE TRAITEMENT DU CANCER OU DE L'AMYLOÏDOSE

(30) Priority: 19.06.2015 US 201562181910 P
(43) Date of publication of application: 25.04.2018
(73) Proprietor: Beijing Artivila BioPharma Co. Ltd., Beijing 102206 (CN)
(72) Inventor: HAN, Guoxia, Philadelphia, PA 19136 (US)
(74) Representative: Serjeants LLP
(86) International application number: PCT/US2016/038350
(87) International publication number: WO 2016/205790

(56) References cited:
- WO-A1-2012/177835
- WO-A1-2014/161072
- WO-A2-2010/114982
- WO-A2-2014/121291
- US-A1- 2005 282 757
- US-A1- 2009 325 903
- BIN YANG ET AL: "A pH-responsive drug nanovehicle constructed by reversible attachment of cholesterol to PEGylated poly(l-lysine) via catechol-boronic acid ester formation", ACTA BIOMATERIALIA, vol. 10, no. 8, 27 May 2014 (2014-05-27), pages 3686-3695, XP055572854, AMSTERDAM, NL ISSN: 1742-7061, DOI: 10.1016/j.actbio.2014.05.018
- HAN LI-QIANG ET AL: "Urea-containing peptide boronic acids as potent proteasome inhibitors", EUROPEAN JOURNAL OF MEDICINAL CHEMISTRY, EDITIONS SCIENTIFIQUE ELSEVIER, PARIS, FR, vol. 125, 14 October 2016 (2016-10-14), pages 925-939, XP029842458, ISSN: 0223-5234, DOI: 10.1016/J.EJMECH.2016.10.023

## Description

### FIELD OF THE INVENTION

The invention herein is directed to chiral specific boron-containing compounds, such as boronate, boronate esters, boranamines, boranediamines, boranamine thioesters, and boronic mono/di-thioesters, as anti-cancer or anti-amyloidosis agents, as well as their compositions, methods of preparation, and methods of treatment.

### BACKGROUND OF THE INVENTION

It is known that some peptide boronic acid compounds are proteasome inhibitors, and they represent an important strategy in cancer therapy. Besides the marketed drug VELCADE^{®} (Bortezomib or chemical name as [(1*R*)-3-methyl-1-[[(2*S*)-1-oxo-3-phenyl-2[(pyrazinylcarbonyl) amino]propyl]amino]butyl] boronic acid) for the treatment of multiple myeloma, other types of cancers, and amyloidosis. There are a number of structurally different peptide boronic acids at various stages of clinical trials. One major issue of this group of boronic acids as (active pharmaceutical ingredient (API) is that they are not very stable at ambient conditions. The drug VELCADE^{®}, for example, needs to be stored at below ambient temperature and kept away from light. Partially related to its instability, the drug VELCADE^{®} is formulated as an injection mixture, and patients must go to doctors' offices or hospitals for drug administration. Boronic acids are known to form many boronic acid derivatives, such as boronates and boronate esters. Boronates and boronate esters are able to be converted back to boronic acid under certain conditions. WO2014/121291A2 discloses a cyclic ester of a boropeptide and a pharmaceutical composition comprising the same. WO2010/114982A2 discloses a cyclic ester of a boropeptide and its use for the treatment of cancer. WO2014/161072A1 discloses a cyclic ester of a boropeptide and its use for the treatment of cancer.

The present invention describes a group of boron-containing compounds, such as boronate, boronate esters, boranamines, boranediamines, boranamine thioesters, and boronic mono/di-thioesters, that are stable at ambient conditions. They can be formulated as both liquid and solid drugs. Therefore, the bioactive component of the drug can be circulated longer in the human body and reach more areas unavailable to the direct injection into blood vessels.

### DEFINITION OF TERMS

Unless otherwise explicitly stated, R is intended to refer an aliphatic or aromatic group.

Unless otherwise explicitly stated, the term "aliphatic" or "aliphatic group" is intended to refer a substituted or unsubstituted straight-chain, branched or cyclic C₁-C₁₂ hydrocarbon, which is completely saturated or which contains one or more units of unsaturation, but which is not aromatic. Aliphatic group also includes atoms other than C in the backbone, such as, but not limited to, N, S, and O. Aliphatic group also includes one or more functional groups attached to the backbone, such as, but not limited to, -OH, -SH, -CO-OH, -CO-OR (ester), -NH₂, -NHR,-CO-R (ketone), -O-SO₂ (sulfonate), -NH-SO₂ (sulfonamide) and -NR-SO₂ (sulfonamide).

Unless otherwise explicitly stated, the term "aromatic" or "aromatic group" is intended to refer a substituted or unsubstituted planar unsaturated ring of atoms that is stabilized by an interaction of the bonds forming the ring. Atoms forming an aromatic group include, but are not limited to, C, O, S, and N.

Unless otherwise explicitly stated, the term "peptide" is intended to refer to a short chain of amino acid monomers linked by amide (-NH-CO- or -NR-CO-) bonds. R represents an aliphatic or aromatic group. Each C atom in the amino acid monomer may have one or two substitution groups, and the substitution groups are aliphatic, aromatic or both aliphatic and aromatic. The covalent chemical bonds are formed when the carboxyl group of one amino acid reacts with the amino group of another.

Unless otherwise explicitly stated, the term "backbone" is intended to refer to the longest hydrocarbon chain in a molecule or a group. It also refers to the longest hydrocarbon chain with other atoms in the chain, such as, but not limited to, O, S, and N, in a molecule or a group.

Boronate, boronate esters, boranamines, boranediamines, boranamine thioesters and boronic mono/di-thioesters are illustrated by the corresponding general structures showed below.

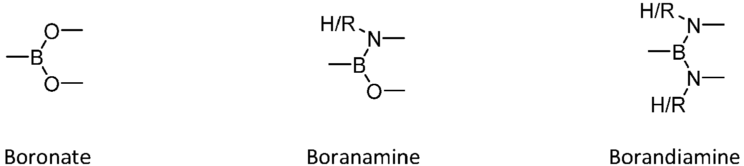

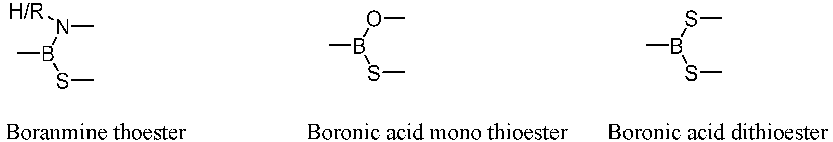

In the compounds of this invention any atom not specifically designated as a particular isotope is meant to represent any stable isotope of that atom. Unless otherwise stated, when a position is designated specifically as "H" or "hydrogen", the position is understood to have hydrogen at its natural abundance isotopic composition. Also, unless otherwise stated, when a position is designated specifically as "D" or "deuterium", the position is understood to have deuterium at an abundance that is at least 3000 times greater than the natural abundance of deuterium, which is 0.015% (i.e., at least 45% incorporation of deuterium).

### SUMMARY OF THE INVENTION

The present invention provides novel boron-containing compounds and stable pharmaceutically acceptable compositions comprising them. These compounds are useful in treatment of diseases, such as cancers, cancerous conditions, plaque, or a plaque-forming condition such as amyloidosis, in a human or warm-blooded animal or mammal.

In one aspect of the invention, a compound of the formula (X-2), or a pharmaceutically acceptable salt thereof, is provided.

In another aspect of the invention, a pharmaceutical composition comprises a compound of formula (X-2), or a pharmaceutically acceptable crystalline form thereof, suitable for the production of a drug in solid and/or liquid formulation.

Non-toxic, pharmacologically acceptable salts useful herein comprise acid addition salts formed with inorganic or organic acids. Examples of suitable such salts include hydrohalides such as hydrochlorides, sulfates, hydrogen sulfates, phosphates, hydrogen phosphates, tartrates, succinates, maleates, benzoates, acetates, propionates, lactates, ascorbinates, and the like.

The compound of formula (X-2) according to the invention can be converted into its non-toxic, pharmacologically acceptable acid addition salts in conventional manner. Acids suitable for salt formation include, for example, hydrochloric acid, hydrobromic acid, hydriodic acid, hydrofluoric acid, sulfuric acid, phosphoric acid, nitric acid, acetic acid, propionic acid, butyric acid, caproic acid, valeric acid, oxalic acid, malonic acid, succinic acid, maleic acid, fumaric acid, lactic acid, tartaric acid, citric acid, malic acid, benzoic acid, p-hydroxybenzoic acid, p-aminobenzoic acid, phthalic acid, cinnamic acid, salicyclic acid, ascorbic acid, methanesulfonic acid, chlorotheophyllin, and the like.

The compound of formula (X-2), or a pharmaceutically acceptable salt thereof, can be incorporated, optionally in combination with other active ingredients, into the usual pharmaceutical preparations such as tablets, coated tablets, capsules, powders, suppositories, or solutions. Such preparations may be produced with use of conventional pharmaceutical excipients, carriers, disintegrants, or lubricants or substances for obtaining delayed or sustained release. The single dose for adults is from about 0.1 to 80 mg (from about 0.0013 to 1.07 mg/kg), preferably, however, from about 1 to 30 mg (from about 0.013 to 0.40 mg/kg), 1 to 4 times daily.

Dependent upon the type and body weight of the patient to be treated, on the type and severity of the disease, on the type of preparation, and on the route of administration, which may be peroral, parenteral, or rectal, as well as on the period or interval over which the administration takes place, it may, however, be necessary to deviate from the above dosages. Thus, it may be sufficient in some cases to administer less than the above-mentioned amount of active ingredient or in some cases the amount may be exceeded. The optimum dosage and route of administration of the active ingredients which are necessary in each case can easily be determined by one skilled in the art.

One aspect of the invention is directed to a compound of the formula: 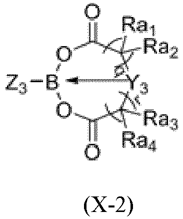 or a pharmaceutically acceptable salt, solvate, and/or hydrate, polymorph crystal or amorphous forms, thereof,
wherein:
Z₃ represents a structure of formula (XI-1) or formula (XI-3): 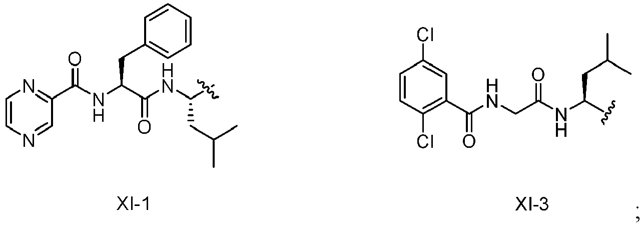
Y₃ is NR;
R represents an aliphatic or aromatic group with or without heteroatom or functional group, such as COOH, OH, or NH₂;
each Ra₁ and Ra₂ independently is H, aliphatic, or aromatic substitution group;
each Ra₃ and Ra₄ independently is H, aliphatic, or aromatic substitution group;
q is 0, 1, or 2; and
r is 0, 1, or 2.

In one aspect of the invention, Z₃ is characterized by the bare bones structure or its derivatives of formula (XI-1):

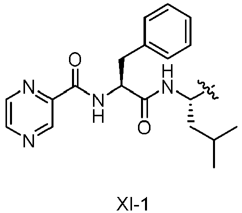

In another aspect of the invention, Z₃ is characterized by the bare bones structure or its derivatives of formula (XI-3):

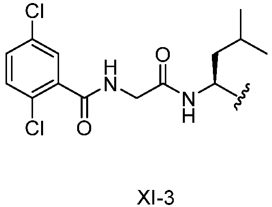

The compound may be one of:

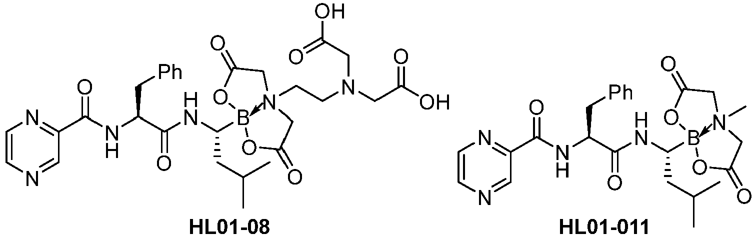

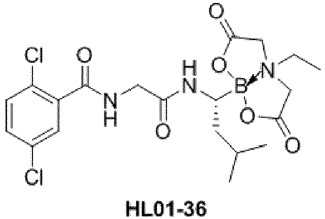

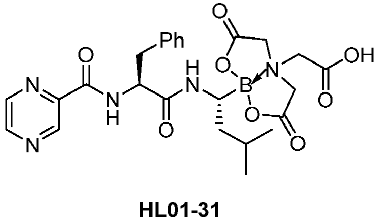

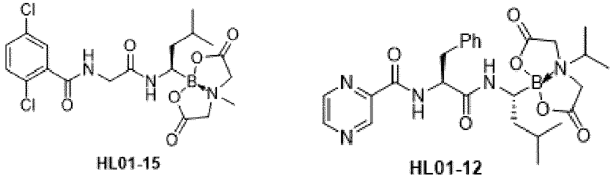

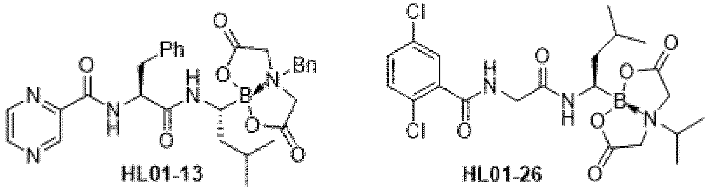

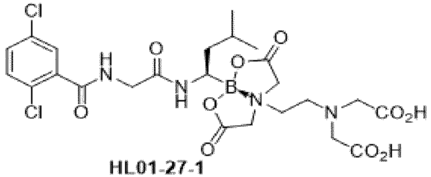

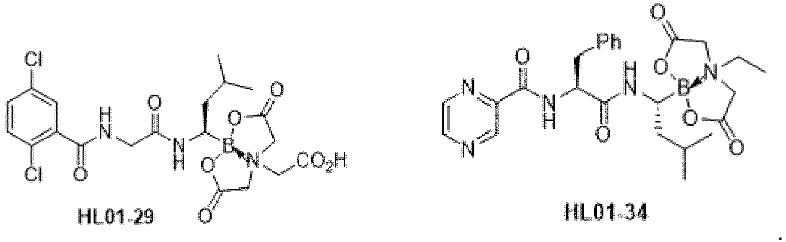

In another aspect of the invention, a composition for use in the treatment of cancer, a cancerous condition, or amyloidosis in a human or animal comprises as active ingredient a pharmaceutically effective amount of a compound of formula (X-2), or a pharmaceutically acceptable acid addition salt thereof, in combination with pharmaceutically acceptable carriers, diluents, or excipients. The cancerous condition or amyloidosis may be multiple myeloma or amyloidosis.

### BRIEF DESCRIPTION OF THE DRAWING

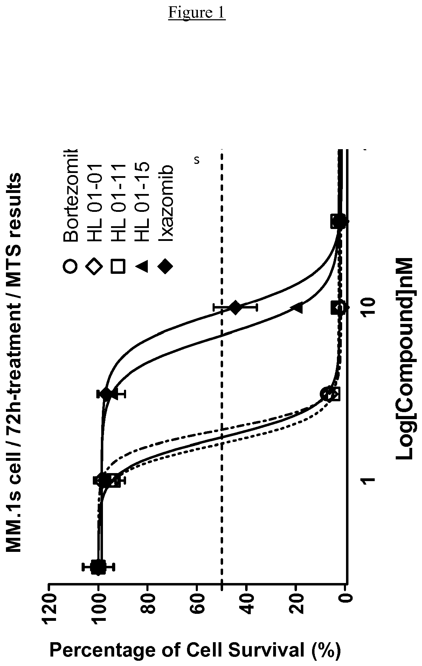

Figure 1 is a graph that represents the results of testing to demonstrate the cytotoxic effects of certain compounds, including compounds prepared according to the invention.

### DETAILED DESCIPTION OF THE INVENTION

The invention can perhaps be appreciated better from the description below, where compounds of the invention have been prepared and then some testing is described.

### EXAMPLES

Boronic acid can readily form oligomeric anhydrides by dehydration of the boronic acid moiety. Hence, the monomeric structure form drawn below, such as HL01-0 drawn in the Example 1, is expressly intended to represent the free boronic acid, oligomeric anhydrides, including, but not limited to, dimers, trimers, and tetramers, and mixtures thereof.

Abbreviations: Bortezomib (HL01-0), Tetrahydrofuran (THF), Ethyl Acetate (EtOAc or EA), N,N-Dimethylsulfoxide (DMSO), Ethylenediaminetetraacetic Acid (EDTA), Hydrogen Chloride (HCl), room temperature (RT), Nuclear Magentci Resonance (NMR), Reverse Phase High Performance Liquid Chromatography (RP-HPLC), Liquid Chromatography-Mass Spectroscopy (LC-MS)

General Characterization Methods: ¹H NMR, ¹³C NMR, HPLC, LCMS.

The product HL01-01 of Example 1 for reference only, characterized by formula HL01-01-1, HL-01-01-2, HL-01-01-3, HL-01-01-4, HL-1-01-5, or a mixture thereof.

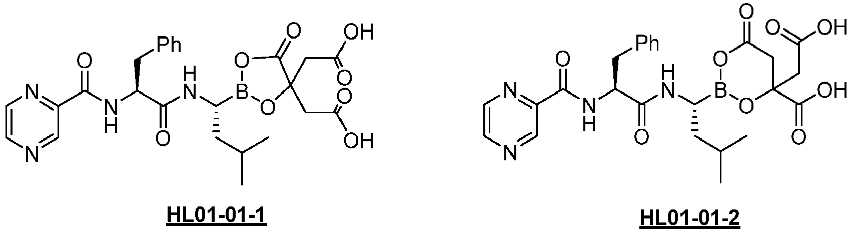

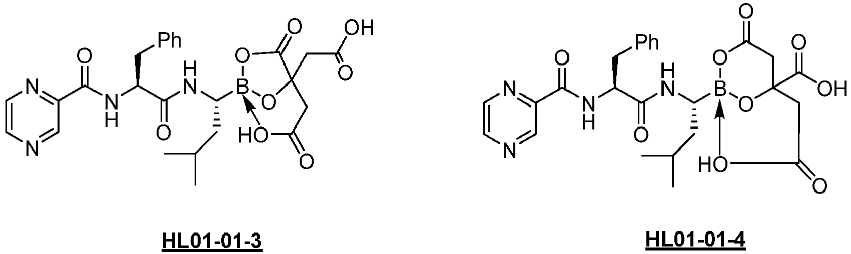

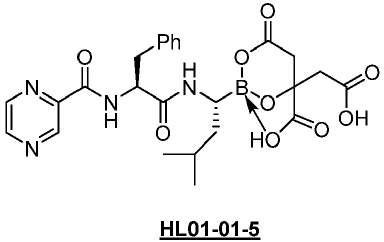

### Example 1

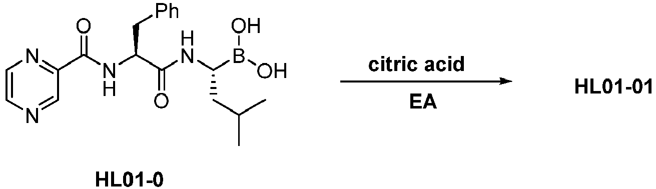

Bortezomib (HL01-0, 1.0 g) was dissolved in EA (80 mL) at 85°C, and citric acid (525 mg, 2.73 mmol) was added under inert gas. The reaction mixture was stirred at 85°C for another 3.5 hrs, then cooled to room temperature (RT), and n-Hexane (24 mL) was added. The white precipitate was filtered and dried to get the white solid HL-01-01. ¹H NMR (d₆-DMSO, 400 MHz) δ 12.08 (br, 1H), 10.62 (br, 1H), 9.19 (br, 1H), 9.18 (s, 1H), 8.89 (s, 1H), 8.77 (s, 1H), 7.28-7.19 (m, 5H), 5.06 (m, 1H), 3.25 (d, 2H, J = 6.8 Hz), 2.85 (m, 1H), 2.72 (m, 2H), 2.57 (m, 2H), 1.45 (m, 1H), 1.10 (m, 1H), 0.97 (m, 1H), 0.78 (d, 6H, J = 4.4 Hz). ¹³C NMR (d₆-DMSO, 400 MHz) δ 177.82, 170.73, 170.62, 163.28, 147.84, 143.98, 143.71, 143.39, 129.28, 128.31, 126.79, 76.24, 50.31, 36.25, 24.55, 23.51, 21.38.

### Example 5

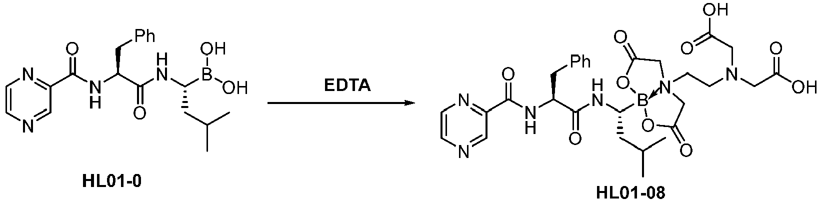

HL01-0 (19.2 mg) was dissolved in DMSO (1.5 mL), and EDTA (7.3 mg, 0.05 mmol) was added under inert gas. The reaction mixture was stirred at 100°C for 3 hrs, then was cooled to RT. The white precipitate was collected under reduced pressure, and dried get the white solid. MS (*m*/*z*): [M+H] calculated for C₂₉H₃₈BN₆O₁₀, 641.46; found, 641.30.

### Example 7

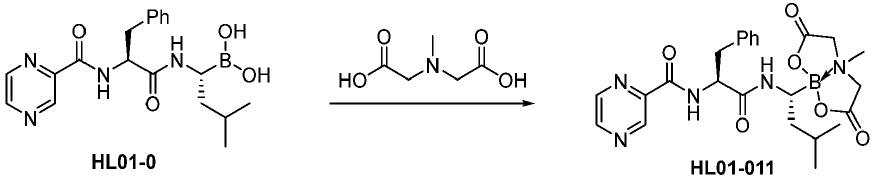

HL01-0 (200 mg) was dissolved in DMSO/EA (6 mL/6 mL ), and N-methyliminodiacetic acid 133 mg, 0.52 mmol) as added under inert gas. The reaction mixture was stirred at 85°C for 5 hrs, and then was cooled to RT. The white precipitate was collected under reduced pressure, and dried to get the white solid. ¹H NMR (d₆-DMSO, 400 MHz) δ 9.07 (d, 1H, J = 1.2 Hz), 8.92 (d, 1H, J = 4.4 Hz), 8.87 (d, 1H, J = 2.4 Hz), 8.74 - 8.73 (m, 1H), 7.30 - 7.23 (m, 5H), 7.20 - 7.15 (m, 1H), 4.70 - 4.64 (m, 1H), 4.22 (d, 1H, J = 17.2 Hz), 4.07 (d, 1H, J = 16.8 Hz), 3.98 (d, 1H, J = 16.8 Hz), 3.57 - 3.51 (m, 2H), 3.09 (s, 1H), 3.07 (d, 1H, J = 2.0 Hz), 2.82 (s, 3H), 1.59 - 1.53 (m, 1H), 1.43 - 1.36 (m, 1H), 1.22 - 1.16 (m, 1H), 0.84 (dd, 6H, J = 11.2, 6.4 Hz). MS (m/z): [M+H] calculated for C₂₄H₃₁BN₅O₆, 496.34; found, 496.20.

### Example 8

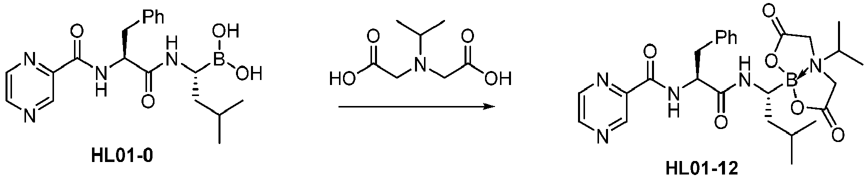

HL01-0 (150 mg) was dissolved in DMSO (5 mL), and N-isopropyl iminodiacetic acid (HL01-12-0, 69 mg, 0.39 mmol) as added under inert gas. The reaction mixture was stirred at 85°C for 5 hrs, and then was cooled to RT. The solvent was removed under reduced pressure to get colorless oil. ¹H NMR (d₆-DMSO, 400 MHz) δ 9.09 (d, 1H, J = 1.6 Hz), 8.91 (d, 1H, J = 8.4 Hz), 8.86 (d, 1H, J = 2.4 Hz), 7.44 (d, 1H, J = 9.6 Hz), 7.30 - 7.16 (m, 5H), 4.68 - 4.62 (m, 1H), 4.28 (d, 1H, J = 18.0 Hz), 3.89 (dd, 2H, J = 18.4, 5.2 Hz), 3.71 - 3.61 (m, 3H), 3.09 (s, 1H), 3.07 (d, 1H, J = 2.8 Hz), 1.61 - 1.55 (m, 1H), 1.49 - 1.42 (m, 1H), 1.16 (dd, 6H, J = 6.0, 2.0 Hz), 0.99 (d, 1H, J = 6.8Hz), 0.84 (dd, 6H, J = 29.2, 6.8 Hz). MS (m/z): [M+H] calculated for C₂₆H₃₅BN₅O₆, 524.4; found, 524.30.

### Example 9

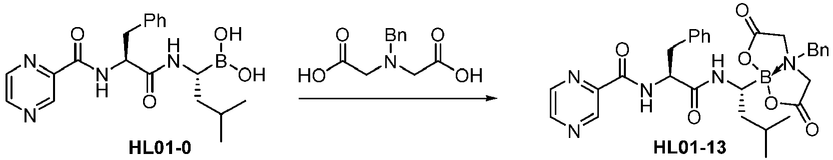

HL01-0 (200 mg) was dissolved in DMSO (3 mL), and N-benzyl iminodiacetic acid (HL01-13-0, 122 mg, 0.547 mmol) as added under inert gas. The reaction mixture was stirred at 85°C for 2 hrs, then was cooled to RT. The solvent was removed under reduced pressure to get colorless oil. ¹H NMR (d₆-DMSO, 400 MHz) δ 9.12 (d, 1H, J = 1.6 Hz), 9.06 (d, 1H, J = 8.0 Hz), 8.87 (d, 1H, J = 2.0 Hz), 8.75 (dd, 1H, J = 2.4, 1.6 Hz), 7.63 - 7.61 (m, 1H), 7.52 - 7.50 (m, 2H), 7.34 - 7.17 (m, 7H), 4.66 - 4.60 (m, 1H), 4.465 (d, 1H, J = 13.2 Hz), 4.26 (d, 1H, J = 13.2 Hz), 4.18 (d, 1H, J = 17.2 Hz), 3.85 - 3.77 (m, 2H), 3.68 - 3.63 (m, 1H), 3.15 - 3.00 (m, 3H), 1.66 - 1.60 (m, 1H), 1.56 - 1.45 (m, 1H), 1.35 - 1.26 (m, 1H), 0.89 (dd, 6H, J = 11.2, 6.8 Hz). MS (m/z): [M+H] calculated for C₃₀H₃₅BN₅O₆, 572.44; found, 572.30.

### Example 13

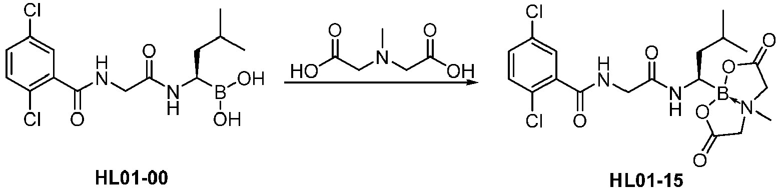

HL01-00 (30 mg, 0.083 mmol) was dissolved in DMSO (2 mL), and N-Methyl iminodiacetic acid (12.2 mg, 0.083 mmol) was added under inert gas. The reaction mixture was stirred at 85°C for 2 hrs, then was cooled to RT. The solvent was removed under reduced pressure and the residue purified by Prep-HPLC to get the white solid, HL01-15 (15 mg, 38.5 %) ¹H NMR (d6-DMSO, 400 MHz) δ 8.84 (s, 1H), 7.54 - 7.52 (m, 3H), 7.33 (d, 1H, J = 8.8 Hz), 4.26 - 4.13 (m, 3H), 4.02 - 3.80 (m, 4H), 2.87 (s, 3H), 1.53 - 1.52 (m, 1H), 1.41 - 1.35 (m, 1H), 1.20 - 1.16 (m, 1H), 0.85 (m, 6H). MS (m/z): [M+H] calculated for C₁₉H₂₅BCl₂N₃O₆, 472.12; found, 472.00.

### Example 24

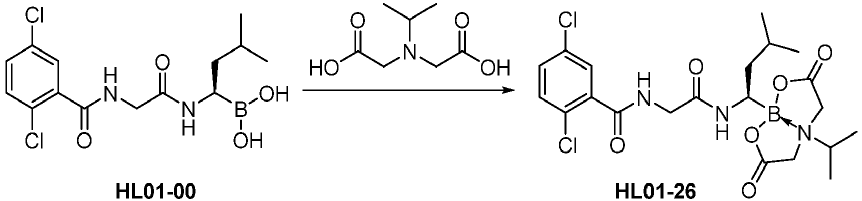

HL01-00 (30mg) was dissolved in DMSO (1mL) at RT, and N-isopropyl iminodiacetic acid (14.53mg, 0.083mmol) was added. The reaction mixture was stirred at 98°C under inert gas for another 6 hrs, then cooled to RT. The solvent was removed to get solid. ¹H NMR (d₆-DMSO, 400 MHz) δ 8.78 (s, 1H), 7.68 (s, 1H), 7.49-7.47 (d, 3H, J=8Hz), 4.32-4.28 (d, 1H, J=16.8Hz), 4.05-3.88 (m, 4H), 3.74-3.67 (m, 3H), 1.54 (s,1H), 1.44-1.38 (t, 2H), 1.23-1.06 (m, 7H), 0.86-0.82 (d, 6H, J=15.2Hz). MS (m/z): [M+H] calculated for C₂₁H₂₉BCₗ₂N₃O₆, 501.18; found, 502.1

### Example 25

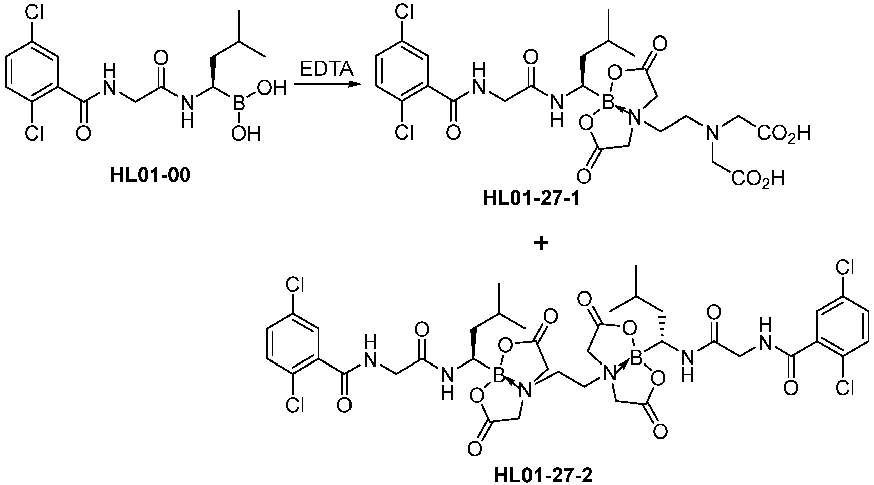

HL01-00 (30mg) was dissolved in DMSO (1mL) at RT, and EDTA (24.23mg, 0.083mmol) was added. The reaction mixture was stirred at 98°C under inert gas for another 6 hrs, then cooled to RT. The solvent was removed to get solid which was purified by RP-HPLC to obtain **HL01-27-1** and dimer **HL01-27-2. HL01-27-1**
MS (*m*/*z*): [M+H] calculated for C₂₄H₃₂BCl₂N₄O₁₀, 618.24; found, 618.4; **HL01-27-2**
MS (*m*/*z*): [M+H] calculated for C₃₈H₄₇B₂Cₗ₄N₆O₁₂, 943.24; found: 943.4

### Example 27

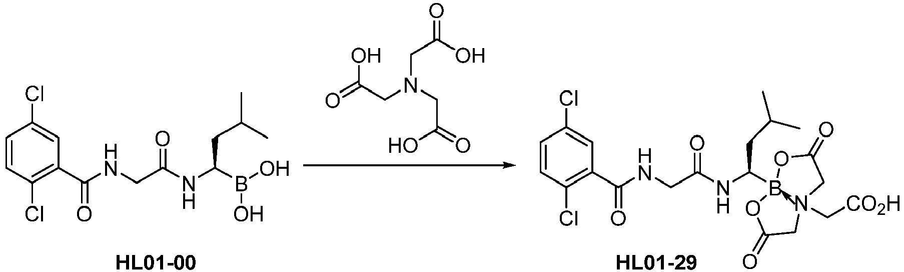

HL01-00 (30mg) was dissolved in DMSO (1mL) at RT, and Nitrilotriacetic acid (15.85mg, 0.083mmol) was added. The reaction mixture was stirred at 85°C under inert gas for another 5 hrs, then cooled to RT. The solvent was removed to get solid. ¹H NMR (d₆-DMSO, 400 MHz) δ 8.81-8.78 (t, 1H), 7.69-7.68 (t, 1H), 7.50-7.38 (m, 3H), 4.43-4.38 (d, 1H,J=20 Hz), 4.30-4.25 (d, 1H,J=16.8 Hz), 4.21-4.00 (m, 4H), 3.91-3.86 (dd, 1H,J=5.2Hz, 15.6Hz), 3.68-3.63 (dd, 1H, J=6.4Hz, 16Hz), 3.59-3.53 (m, 1H), 1.56-1.53 (m, 1H), 1.39-1.36 (m, 1H), 1.21-1.17 (m, 1H), 0.85 (dd, 6H, J=6.4Hz, 14Hz). MS (*m*/*z*): [M+H] calculated for C₂₀H₂₅BCl₂N₃O₈, 517.14; found, 517.3.

### Example 29

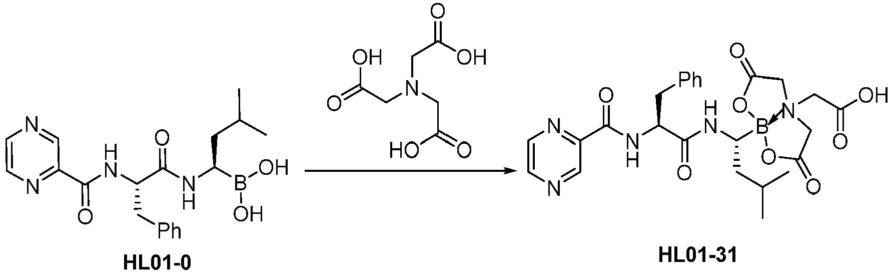

HL01-0 (30mg) was dissolved in DMSO (1mL) at RT, and Nitrilotriacetic acid (14.89mg, 0.078mmol) was added. The reaction mixture was stirred at 85°C under inert gas for another 6 hrs, then cooled to RT. The solvent was removed to get solid. ¹H NMR (d₆-DMSO, 400 MHz) δ 9.07-9.06 (d, 1H, J=1.2Hz), 8.92-8.83 (m, 2H), 8.74-8.70 (m, 1H), 7.40-7.38 (d, 1H, J=10Hz), 7.29-7.12 (m, 5H), 4.71-4.65 (m, 1H), 4.40-4.04 (m, 4H), 3.55-3.55 (m, 3H), 3.09-3.06 (m, 2H), 1.60-1.51 (m, 1H), 1.45-1.38 (m,1H), 1.23-1.17 (m, 1H),0.84 (dd, 6H, J=6.4Hz, 18.4Hz). MS (m/z): [M+H] calculated for C₂₅H₃₁BN₅O₈, 540.35; found, 540.3

### Example 32

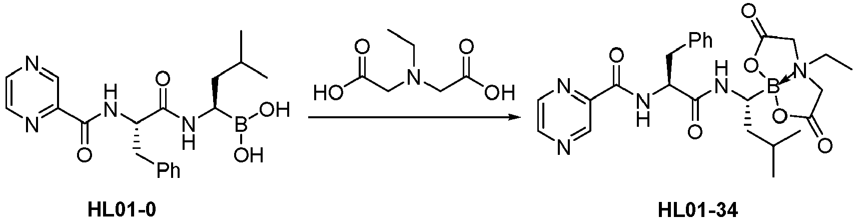

HL01-0 (30mg) was dissolved in DMSO (1mL) at RT, and N-ethyl-iminodiacetic acid (12.57mg, 0.078mmol) was added. The reaction mixture was stirred at 85°C under inert gas for another 5 hrs, then cooled to RT. The solvent was removed to get solid. ¹H NMR (d₆-DMSO, 400 MHz) δ 9.12-9.08 (m 1H), 8.93-8.84 (m, 2H), 8.74-8.66 (m, 1H), 7.35-7.12 (m, 5H), 4.93-4.87 (q, 1H), 4.81-4.75 (q, 1H), 4.71-4.65 (q, 1H), 4.18-3.93 (m, 4H), 3.30-3.24 (q, 1H), 3.14-3.03 (m, 2H),1.56-1.49 (m, 1H), 1.41-1.25 (m, 2H), 1.22-1.13 (m, 3H), 0.86-0.74 (m, 6H).

### Example 34

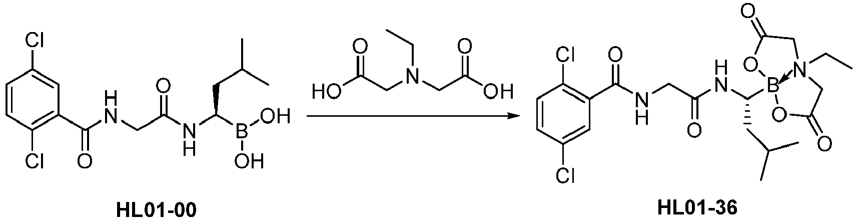

HL01-00 (30mg, 0.078 mmol) was dissolved in DMSO (1mL) at RT, and N-ethyl-iminodiacetic acid (12.57mg, 0.078mmol) was added. The reaction mixture was stirred at 85°C under inert gas for another 5 hrs, then cooled to RT, The solvent was removed to get solid. ¹H NMR (d₆-DMSO, 400 MHz) δ8.78-8.66 (dt, 1H), 7.69 (s, 1H), 7.52-7.44 (m, 2H), 7.35-7.29 (m, 1H),4.19-3.82 (m, 6H), 3.18-3.09 (m, 2H), 2.99(s, 1H), 1.61-1.43 (m, 1H), 1.38-1.28 (m, 2H), 1.20-1.11 (m, 3H),0.87-0.82 (m, 6H).

### Stability Study

Compounds were tested for their stability, under three different conditions (see Table 1), by the following procedures:
1. Room Temperature and Dry: compounds were left at the room temperature (20°C) under inert dry gas;
2. 30 °C and 65% Humidity: compounds sealed with double bags were put into a stability chamber. Temperature was set at 30°C while the humidity was controlled at 65%.
3. 37 °C pH=7.4 solution: compounds were dissolved in PBS solution and then the entire solution was submerged under a 37°C water bath.

**Table 1. Stability study of designed chiral specific boron derivatives.**

| **Compound** | **Room Temp Dry** | **30°C/ 65% Humidity** | **37°C H₂O/ pH7.4** |
|---|---|---|---|
| HL01-0 | - | | |
| HL01-11 | + | + | + |

| | | | |
|---|---|---|---|
| Note: "+" meaning stable, "-" meaning quickly degradable, as determined by RP-HPLC. | | | |

### Cell Viability Assay

### 1. Cell line

The multiple myeloma cell line MM1.S was obtained from the ATCC. Cells were cultured in standard RPMI-1640 media (Hyclone) supplemented with 10% heat-inactivated fetal bovine serum (Gibco), 1% penicillin/streptomycin (Gibco) and grown in a humidified incubator with 5% CO₂ at 37°C.

Eight thousand MM1.S cells per well were seeded in tissue culture-treated 96-well plate (Nunc) and incubated overnight.

### 2. Compound Treatment

Eighteen hours after seeding, MM1.S cells were treated with a series of concentrations of five different compounds (Bortezomib, HL01-01, HL01-11, HL01-15, HL01-17 (Ixazomib)) or DMSO vehicle control for 72 hrs in a humidified incubator with 5% CO₂. at 37 °C. The concentrations of five different compounds were 0.3, 1, 3.16, 10, 31.6, 100, 316, and 1000nM. Each well had the same DMSO concentration of 0.05%.

### 3. Cell Viability Assay (MTS assay)

The effects of compounds on cell viability were assessed using CellTiter 96^{®}A Queous One solution Reagent (Promega; G3580).

After being treated with various compounds for 72 hrs, cells in the 96-well plate were centrifuged 1000rpm for 3 minutes, and 180ul supernatant were removed from each well. Cells were incubated with 20ul CellTiter 96^{®}A Queous One solution Reagent and put into incubator with 5% CO₂. at 37 °C for an additional 4 hrs. The absorbance was measured at 490nm with Automatic Microplate reader (Infinite M1000 pro, Tecan). There was a liner relationship (r²=0.99) between absorbance and cell number in each plate format. Four sets of experiments for each drug combinations were carried out. Cell viability (Percentage of Cell Survival) was calculated by the following formula: cell viability (%) =(average absorbance of treated group-average absorbance of blank) / (average absorbance of untreated group - average absorbance of blank) × 100%. IC50 values were calculated using Prism software.

### Results

The cytotoxic effects of compounds including Bortezomib and HL01-11on MM1.S cell were assessed using the MTS assay. It was found that all compounds could dose-dependently decrease the cell viability in MM1.S cell. As represented by the cell line assay shown in Fig 1. Bortezomib and HL01-11 exhibited strong cytotoxic effects on MM1.S cells and both compounds had similar IC₅₀ values nearly 2 nM.

**Table 2**

| **Compound** | **IC₅₀ (nM)** |
|---|---|
| Bortezomib | 1.76 |
| HL01-01 | 1.96 |
| HL01-11 | 1.62 |

Five compounds exhibited cytotoxicity in MM1.S cells. MM1.S cells were treated with various concentrations of Bortezomib and HL01-11 for 72 h. Cell viability was assessed using the MTS assay. Mean ± SEM (n=4).

## Claims

1. A compound of the formula (X-2): 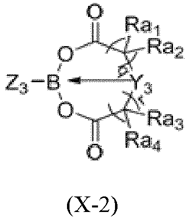 or a pharmaceutically acceptable salt, solvate, hydrate, polymorph crystal or amorphous forms, thereof, wherein:
Z₃ represents a structure of formula (XI-1) or formula (XI-3): 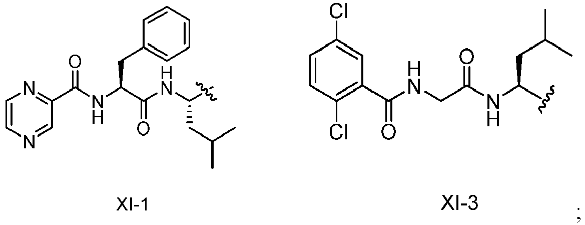
Y₃ is NR;
R represents an aliphatic or aromatic group with or without heteroatom or functional group, such as COOH, OH, or NH₂;
each Ra₁ and Ra₂ independently is H, aliphatic, or aromatic substitution group;
each Ra₃ and Ra₄ independently is H, aliphatic, or aromatic substitution group;
q is 0, 1, or 2; and
r is 0, 1, or 2.

2. The compound of claim 1, wherein said compound is one of:

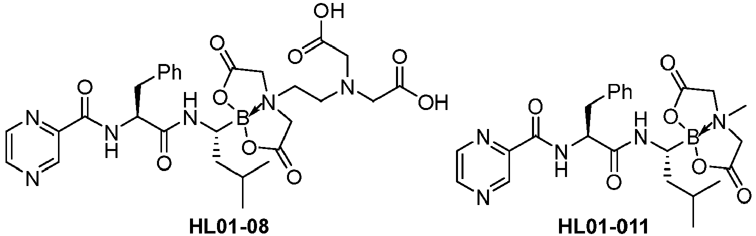

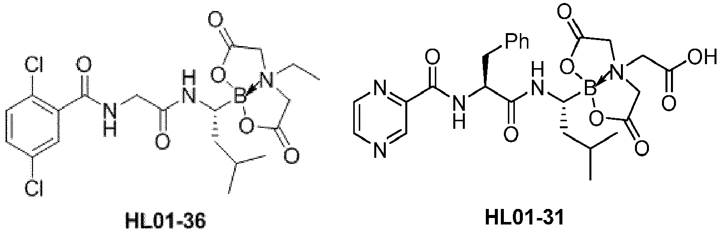

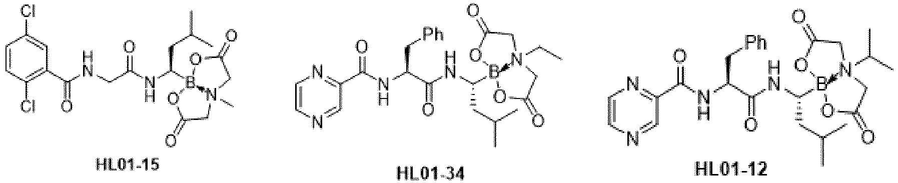

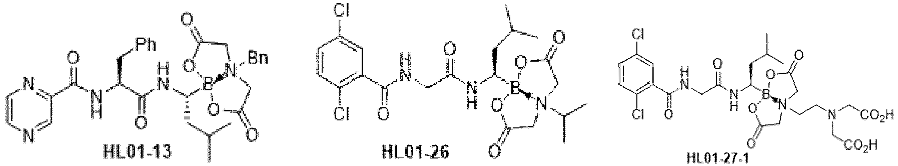

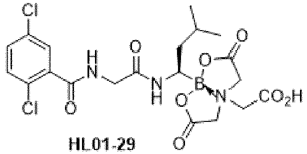

3. A composition for use in the treatment of cancer, a cancerous condition, or amyloidosis in a human or mammal, comprising as active ingredient a pharmaceutically effective amount of a compound of formula (X-2) of claim 1, or a pharmaceutically acceptable acid addition salt thereof, in combination with pharmaceutically acceptable carriers, diluents, or excipients.

4. The composition for use of claim 3, wherein the cancerous condition or amyloidosis is multiple myeloma or amyloidosis.

## Patentansprüche

1. Verbindung der Formel (X-2): 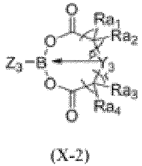 oder ein pharmazeutisch verträgliches Salz, Solvat, Hydrat, ein pharmazeutisch verträglicher polymorpher Kristall oder amorphe Formen davon, wobei:
Z₃ eine Struktur von Formel (XI-1) oder Formel
(XI-3) darstellt: 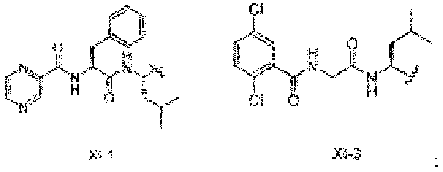
Y₃ NR ist;
R eine aliphatische oder aromatische Gruppe mit oder ohne Heteroatom oder funktionelle Gruppe wie beispielsweise COOH, OH oder NH₂ darstellt;
Ra₁ und Ra₂ jeweils unabhängig voneinander H, eine aliphatische oder aromatische Substitutionsgruppe sind;
Ra₃ und Ra₄ jeweils unabhängig voneinander H, eine aliphatische oder aromatische Substitutionsgruppe sind;
q 0, 1 oder 2 ist; und
r 0, 1 oder 2 ist.

2. Verbindung nach Anspruch 1, wobei die Verbindung eine ist von:

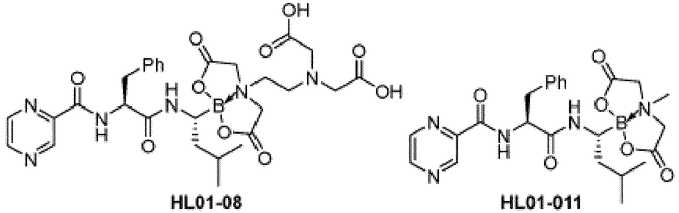

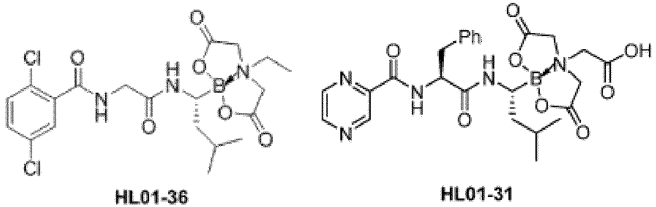

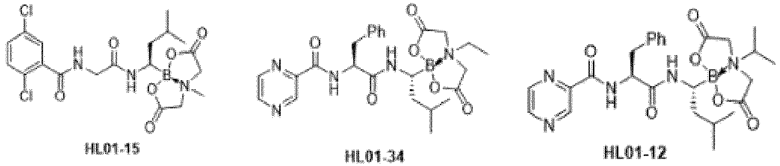

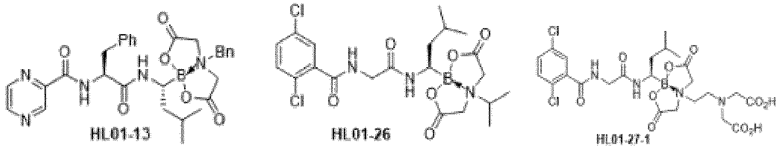

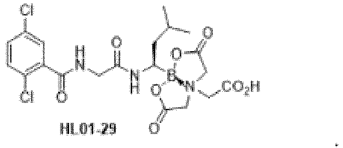

3. Zusammensetzung zur Verwendung bei der Behandlung von Krebs, einer Krebserkrankung oder Amyloidose bei einem Menschen oder Säugetier, umfassend als Wirkstoff eine pharmazeutisch wirksame Menge einer Verbindung von Formel (X-2) nach Anspruch 1 oder eines pharmazeutisch verträglichen Säureadditionssalzes davon, in Kombination mit pharmazeutisch verträglichen Trägern, Verdünnungsmitteln oder Hilfsstoffen.

4. Zusammensetzung zur Verwendung nach Anspruch 3, wobei die Krebserkrankung oder Amyloidose ein multiples Myelom oder eine Amyloidose ist.

## Revendications

1. Composé de la formule (X-2) : 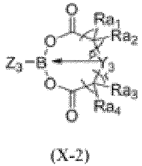 ou sel, solvate, hydrate, cristal polymorphe ou formes amorphes pharmaceutiquement acceptables de celui-ci, dans lequel :
Z₃ représente une structure de formule (XI-1) ou de formule (XI-3) : 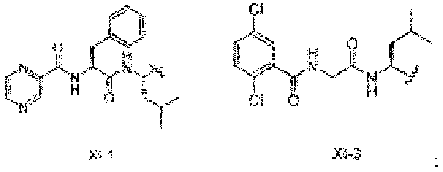
Y₃ est NR ;
R représente un groupe aliphatique ou aromatique avec ou sans hétéroatome ou un groupe fonctionnel, tel que COOH, OH ou NH₂ ;
chaque Ra₁ et Ra₂ est indépendamment H, un groupe de substitution aliphatique ou aromatique ;
chaque Ra₃ et Ra₄ est indépendamment H, un groupe de substitution aliphatique ou aromatique ;
q est 0, 1, ou 2 ; et
r est 0, 1 ou 2.

2. Composé selon la revendication 1, dans lequel ledit composé est l'un de :

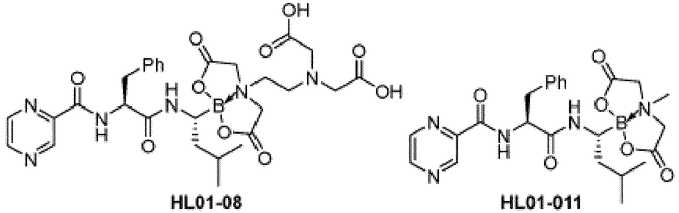

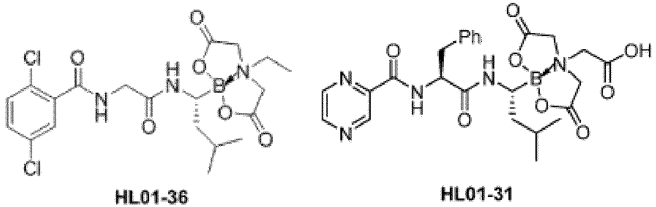

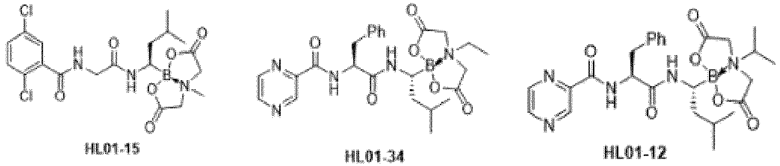

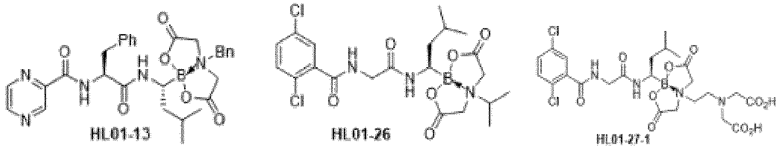

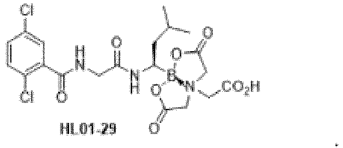

3. Composition destinée à être utilisée dans le traitement du cancer, d'un état cancéreux ou de l'amylose chez un être humain ou un mammifère, comprenant, en tant que principe actif, une quantité pharmaceutiquement efficace d'un composé de formule (X-2) selon la revendication 1, ou un sel d'addition acide pharmaceutiquement acceptable de celui-ci, en combinaison avec des vecteurs, diluants ou excipients pharmaceutiquement acceptables.

4. Composition destinée à être utilisée selon la revendication 3, dans laquelle l'état cancéreux ou l'amylose est le myélome multiple ou l'amylose.
